(19) Europäisches Patentamt
European Patent Office
Office européen des brevets

(11) Numéro de publication : **0 449 731 A1**

# (12) DEMANDE DE BREVET EUROPEEN

(21) Numéro de dépôt : **91400834.7**

(51) Int. Cl.$^5$ : **A61K 31/715**, A61K 47/48

(22) Date de dépôt : **28.03.91**

(30) Priorité : **28.03.90 FR 9003938**

(43) Date de publication de la demande : **02.10.91 Bulletin 91/40**

(84) Etats contractants désignés : **AT BE CH DE DK ES FR GB GR IT LI LU NL SE**

(71) Demandeur : **LABORATOIRES CRINEX**
**3 Rue de Gentilly**
**F-92120 Montrouge (FR)**

(72) Inventeur : **Saleh, Saleh Ismail**
**El Hawatka**
**Assiout (EG)**
Inventeur : **Stamm, André**
**33A rue des Olives**
**F-67210 Griesheim Pres Molsheim (FR)**

(74) Mandataire : **Orès, Bernard et al**
**Cabinet ORES 6, Avenue de Messine**
**F-75008 Paris (FR)**

## (54) Composition pharmaceutique à administration perlinguale, antiinflammatoire et/ou analgésique.

(57) Composition pharmaceutique apte à être administrée par voie perlinguale, notamment sous forme de composition à mâcher, antiinflammatoire et/ou analgésique comprenant un antiinflammatoire non stéroïdien à caractère hydrophobe. Ladite composition comprend au moins en association un complexe d'inclusion entre le kétoprofène et une β-cyclodextrine, éventuellement substituée et un polyol.

EP 0 449 731 A1

La présente invention est relative à une composition pharmaceutique à administration perlinguale, antiinflammatoire et/ou analgésique comprenant un antiinflammatoire non stéroïdien à caractère hydrophobe.

On entend par antiinflammatoire non stéroïdien (AINS) à caractère hydrophobe, notamment des AINS de type propionique, de structure

$$R-\underset{\displaystyle CH_3}{\underset{|}{C}H}-COOH,$$

dans lesquels R comprend au moins un cycle aromatique.

On peut citer notamment comme AINS propionique le flurbiprofène, l'ibuprofène, le fénoprofène, le kétoprofène, le naproxène, le pirprofène ou l'acide tiaprofénique.

Ces différents AINS propioniques, lorsqu'ils sont administrés par voie orale, se présentent généralement sous forme de comprimés ou de gélules et sont insolubles dans l'eau. Cependant, on a constaté que les formes orales solides disponibles (comprimés, gélules) ont l'inconvénient majeur d'accentuer les effets indésirables gastro-intestinaux propres aux AINS, notamment au niveau de la muqueuse gastrique, dans laquelle ils entraînent une libération localisée.

De plus, on a également constaté qu'avec les formes orales solides actuellement décrites, la biodisponibilité du principe actif était faible, notamment en raison d'un effet de premier passage hépatique conduisant à des taux sanguins faibles.

C'est pourquoi la Demanderesse s'est donné pour but de pourvoir à une composition sous forme solide, administrable par voie orale, à base d'AINS et notamment d'AINS propionique qui répond mieux aux nécessités de la pratique, notamment en ce qu'elle ne présente pas les inconvénients des formes orales solides de l'Art antérieur, en particulier en libérant le principe actif rapidement et totalement mais de manière diffuse, c'est-à-dire non concentrée ou localisée sur une zone de la muqueuse gastrique, diminuant ainsi l'irritation locale propre à ces produits.

La présente invention a pour objet une composition pharmaceutique administrable par voie orale, du type comprenant un complexe d'inclusion entre un AINS propionique et une β-cyclodextrine, caractérisée en ce qu'elle comprend au moins en association un complexe d'inclusion entre un AINS propionique tel que le kétoprofène et une β-cyclodextrine, éventuellement substituée et un polyol et en ce qu'elle est apte à être administrée par voie perlinguale.

En effet, les β-cyclodextrines sont des oligo-saccharides cycliques constitués de 7 unités de glucose, plus hydrophiles à l'extérieur qu'à l'intérieur et qui ont la propriété de former des composés d'inclusion avec certaines molécules hydrophobes.

La formation de ces inclusions est fonction de la dimension des molécules associées par rapport à la dimension de la cavité des β-cyclodextrines (0,6 à 0,64 nm).

On peut citer notamment comme β-cyclodextrines substituées, l'hydroxypropyle β-cyclodextrine et la méthyle β-cyclodextrine.

Selon un mode de réalisation avantageux, ladite composition comprend en outre au moins un autre véhicule et/ou un adjuvant pharmaceutiquement acceptable.

Selon un autre mode de réalisation avantageux de ladite composition, le rapport kétoprofène/β-cyclodextrine est compris entre 1:1 et 6:1.

La composition conforme à l'invention apte à être administrée par voie perlinguale, notamment sous forme de composition à mâcher, augmente, de manière inattendue, la biodisponibilité de l'AINS de manière significative tout en masquant le goût amer du kétoprofène.

Elle présente de plus les avantages suivants :

- elle augmente la solubilité des AINS propioniques en milieu aqueux ;
- elle augmente la stabilité (notamment stabilité à la lumière) des AINS propioniques ;
- elle a également l'avantage de ne pas être irritante au niveau de la bouche ;
- de plus, elle permet une libération rapide et totale du principe actif, mais diffuse et ainsi l'obtention d'un pic sanguin en un temps très court, ce qui est particulièrement avantageux pour un AINS à propriétés analgésiques ;
- elle permet notamment une administration perlinguale des AINS propioniques, ce qui évite ainsi l'effet de premier passage hépatique et améliore encore la biodisponiblité de ces derniers ;
- elle est d'utilisation aisée, notamment chez l'enfant ou le sujet âgé ;.
- elle a également l'avantage de diminuer les effets secondaires gastro-intestinaux propres aux AINS.

Conformément à l'invention, le polyol est avantageusement choisi dans le groupe qui comprend le xylitol, le sorbitol, le mannitol, le maltitol, le glucose hydrogéné.

La préparation du composé d'inclusion est réalisée de manière connue en elle-même, par exemple par le mélange d'une β-cyclodextrine appropriée et de kétoprofène, sous forme de poudres, en présence d'une quantité suffisante d'eau pour former une pâte, puis évaporation de l'eau.

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

**Exemple 1 : Préparation d'un complexe kétoprofène-β-cyclodextrine.**

a. Préparation d'une pâte β-cyclodextrine-eau :

– on introduit la β-cyclodextrine (ROQETTE, France) dans un mélangeur, puis on ajoute progressivement une partie d'eau pour trois parties de β-cyclodextrine tout en mélangeant ;

– on continue à mélanger jusqu'à l'obtention d'une pâte homogène (environ 5 minutes).

b. Introduction du kétoprofène à encapsuler :

– on ajoute progressivement, tout en mélangeant, la quantité de kétoprofène à encapsuler (rapport 1:6), puis on mélange encore pendant 5 à 10 minutes.

c. Séchage du composé encapsulé :

On évapore l'eau résiduelle sous vide à 50°C, dans un évaporateur à rotation (BUCHI Rotavapor, Suisse). On peut également sécher ledit mélange par le froid ou bien simplement à l'air chaud.

Le complexe peut également être préparé par simple mélange des poudres.

d. Caractéristiques des complexes d'inclusion obtenus

– Les thermogrammes des mélanges de kétoprofène et de β-cyclodextrine (même rapport molaire) montrent deux pics endothermiques à 54,6°C et 96,54°C correspondant respectivement à la β-cyclodextrine et au kétoprofène. Ces deux pics disparaissent avec le complexe tel que préparé ci-dessus.

– Les profils de diffraction aux rayons X sont également différents pour les mélanges des deux produits et le complexe d'inclusion (formation d'une nouvelle phase solide).

**Exemple 2 : Préparation de comprimés à mâcher.**

– Formule 1 :

| | | |
|---|---|---|
| Complexe (6:1) de kétoprofène-β-cyclodextrine | 350 | mg |
| Mannitol | 14 | mg |
| Stéarate de magnésium | 14 | mg |
| Saccharine sodique | 3,5 | mg |
| Arôme de mandarine | 7 | mg |
| | 724,5 | mg |

pour obtenir des comprimés plats de 12 mm d'épaisseur.

– Formule 2 :

| | | |
|---|---|---|
| Complexe (6:1) de kétoprofène-β-cyclodextrine | 350 | mg |
| Sorbitol cristallin ("NEOSORB") | 350 | mg |
| Stéarate de magnésium | 14 | mg |
| Saccharine sodique | 3,5 | m |
| Arôme de mandarine | 7 | mg |
| | 724,5 | mg |

pour obtenir des comprimés plats de 12 mm d'épaisseur.
– Formule 3 :

| | | |
|---|---|---|
| Complexe (6:1) de kétoprofène-β-cyclodextrine | 350 | mg |
| Mannitol | 800 | mg |
| Stéarate de magnésium | 23 | mg |
| Saccharine sodique | 5,75 | mg |
| Arôme de mandarine | 11,50 | mg |
| Colorant d'Orange S | 0,23 | mg |
| | 1190,48 | mg |

Les formules ci-dessus sont comprimées en utilisant une machine à compresser à un poinçon KORSCH EK/O puis des essais sont réalisés sur les comprimés obtenus (contrôle de l'uniformité de poids, contrôle de dureté, friabilité).

Les comprimés obtenus ont des propriétés organoleptiques remarquables quant au goût et à l'odeur et masquent le goût amer du kétoprofène.

Ils montrent également de bonnes caractéristiques physiques comme visible sur le Tableau I ci-après.

La dureté moyenne obtenue est due à l'extrême sécheresse du complexe utilisé. La dureté pourrait être augmentée en incorporant 10 % d'AVICEL (cellulose micro-cristalline) dans la formule ou bien en utilisant de l'EMDEX comme excipient) la place du mannitol ou du sorbitol dans les formules.

TABLEAU I

| Formule | Poids | | Dureté | Friabilité |
|---|---|---|---|---|
| | moyenne | c.v. % | (kg) | (perte %) |
| 1 | 0,7316 | 1,9 | 4,3 | 1,0 |
| 2 | 0,7227 | 10,00 | 4,8 | 1,2 |
| 3 | 1,2388 | 0,09 | 3,4 | 1,3 |

**<u>Exemple 3</u> : Test de dissolution in vitro.**

Ce test est réalisé en utilisant, par exemple une méthode de la Pharmacopée américaine par agitation à 37°C dans l'eau distillée (100 cycles/ minute). Des échantillons filtrés de 1 ml sont prélevés à des intervalles de temps réguliers, dilués et lus au spectrophotomètre.

Une dissolution rapide fournissant 100 % du contenu en kétoprofène dans les deux minutes (comprimé cassé) est observée. Le temps de dissolution des comprimés à mâcher de kétoprofène conformes à l'invention est très court par rapport à une gélule de kétoprofène du commerce (PROFENID). Les caractéristiques de dissolution sont présentées dans le Tableau II ci-après ainsi qu'à la figure 1.

TABLEAU II

| | % de kétoprofène libéré | | | | |
|---|---|---|---|---|---|
| Temps (min) | Formule 1 | Formule 2 | Formule 3 | Gélule du commerce | Formule1 broyée |
| 2 | 27 | 26 | 26 | 1 | 100 |
| 5 | 42 | 33 | 39 | 2 | 100 |
| 10 | 64 | 50 | 66 | 6 | 100 |
| 15 | 87 | 71 | 87 | 16 | 100 |
| 20 | 96 | 84 | 97 | 27 | 100 |
| 30 | 98 | 99 | 100 | 43 | 100 |
| 45 | 99 | 100 | 100 | 62 | 100 |
| 60 | 100 | 100 | 100 | 71 | 100 |

**<u>Exemple 4</u> : Etude de biodisponibilité des comprimés à mâcher de kétoprofène conformes à l'invention.**

Trois adultes (38, 30 et 30 ans et dont les poids respectifs sont de 75, 60 et 56 kg) ont participé à cet essai.

Après un jeûne d'une douzaine d'heures, on leur a fait avaler soit une gélule de kétoprofène (PROFENID) avec 250 ml d'eau, soit un comprimé à mâcher selon l'invention puis 250 ml d'eau, pour pouvoir recueillir une quantité d'urine suffisante. Ils n'ont pas absorbé d'autre nourriture ou d'autre liquide pendant les 3 heures qui suivent l'administration du médicament.

Un intervalle d'une semaine sépare les deux traitements. (PROFENID et pâte à mâcher)

L'urine est collectée aux temps suivants après l'administration du médicament : t = 0, t = 1 h, t = 2 h, t = 3 h, t = 4 h, t = 6 h, t = 8 h, t = 12 h, et t = 24 h. Les échantillons sont congelés jusqu'au moment de l'analyse.

On dose dans les échantillons d'urine le kétoprofène (libre et conjugué) par une méthode HPLC et sur une colonne à phase inverse (125 x 4,6 mm, LICHROSORB RP8).

Cette étude a montré la supériorité des comprimés à mâcher sur les gélules, par l'obtention d'une excrétion urinaire plus rapide et l'obtention d'une aire sous la courbe reflétant des taux sanguins plus élevés pour la forme comprimé à mâcher.

Le Tableau III ci-après et la figure 1 montrent les caractéristiques comparées des comprimés à mâcher de kétoprofène et des gélules.

TABLEAU III

| GELULE | | | | COMPRIMES A MACHER | | |
|---|---|---|---|---|---|---|
| Temps | Quantité excrétée | Quantités cumulées excrétées | Taux | Quantité excrétée | Quantités cumulées excrétées | Taux |
| (h) | (mg) | (mg) | (mg/h) | (mg) | (mg) | (mg/h) |
| 1 | 5,39 | 5,39 | 5,39 | 8,89 | 8,89 | 8,89 |
| 2 | 7,98 | 13,37 | 7,98 | 10,47 | 19,36 | 10,47 |
| 3 | 5,10 | 18,47 | 5,10 | 8,87 | 28,23 | 8,87 |
| 4 | 3,67 | 22,14 | 3,67 | 2,80 | 30,83 | 2,60 |
| 6 | 3,40 | 25,54 | 1,70 | 2,38 | 33,21 | 1,19 |
| 8 | 1,28 | 26,84 | 0,64 | 2,10 | 35,31 | 1,05 |
| 12 | 1,00 | 27,82 | 0,25 | 1,49 | 36,80 | 0,37 |
| 24 | 1,05 | 28,87 | 0,09 | 1,11 | 37,90 | 0,09 |

La figure 2 qui comporte en abscisse le temps en heures et en ordonnées, le taux d'excrétion urinaire de kétoprofène, montre un taux d'excrétion urinaire plus important pour la forme à mâcher contenant 50 mg de kétoprofène (-.-) que pour les comprimés de PROFENID à 50 mg (-+-).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

**1/** Composition pharmaceutique administrable par voie orale, du type comprenant un complexe d'inclusion AINS propionique/β-cyclodextrine, caractérisée en ce qu'elle comprend au moins en association un complexe d'inclusion entre du kétoprofène et une β-cyclodextrine, éventuellement substituée et un polyol et en ce qu'elle est apte à être administrée par voie perlinguale.

**2/** Composition selon la revendication 1, caractérisée en ce qu'elle comprend en outre au moins un autre véhicule et/ou un adjuvant pharmaceutiquement acceptable.

**3/** Composition selon la revendication 1 ou la revendication 2, caractérisée en ce que le rapport kétoprofène/β-cyclodextrine est compris entre 1:1 et 6:1.

**4/** Composition selon l'une quelconque des revendications 1 à 3, caractérisée en ce que le polyol est avantageusement choisi dans le groupe qui comprend le xylitol, le sorbitol, le mannitol, le maltitol, le glucose hydrogéné.

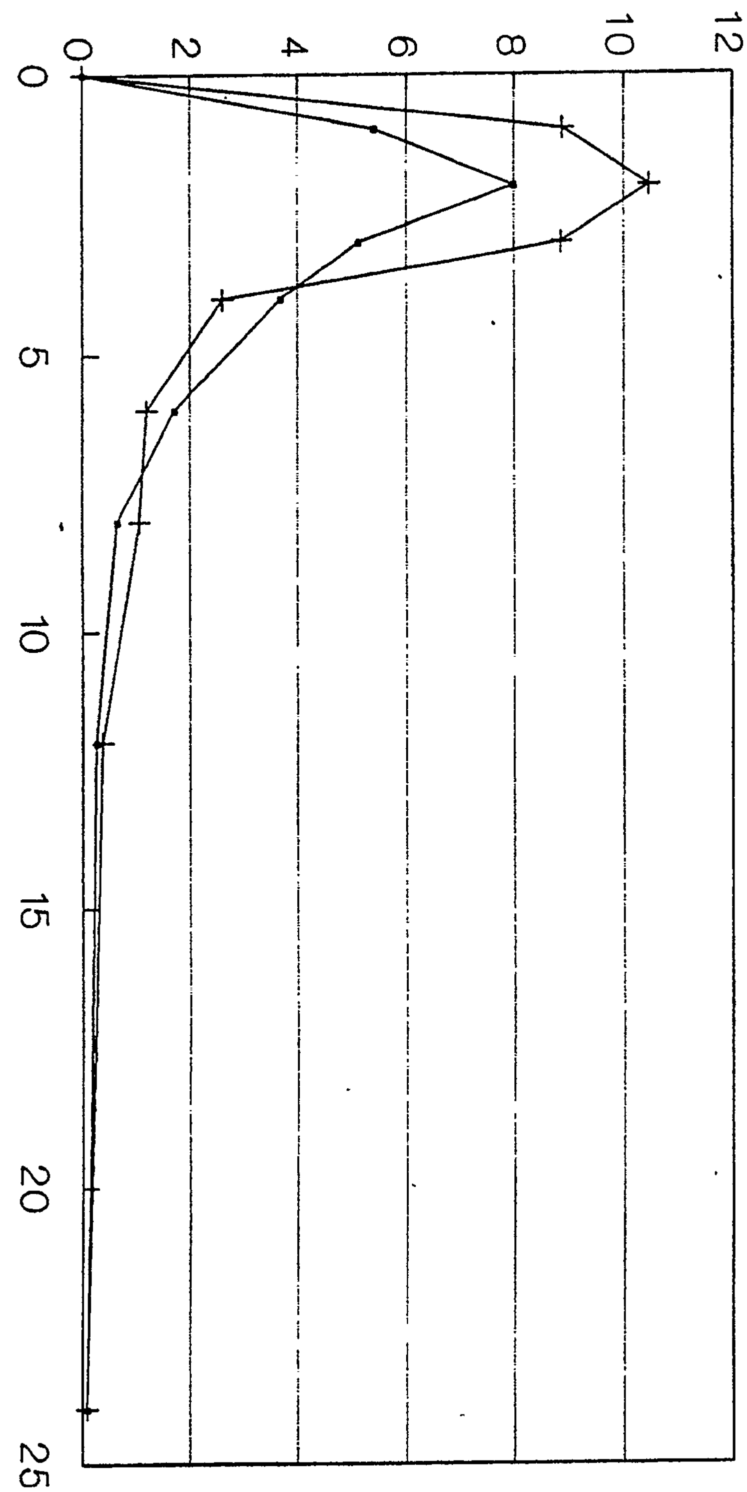


FIGURE 1

Office européen des brevets

# RAPPORT DE RECHERCHE EUROPEENNE

Numero de la demande

EP 91 40 0834

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 346 006 (RECKITT & COLMAN PRODUCTS LTD)<br>* Pages 6-7, exemple 11; page 3, lignes 28-29 *<br>--- | 1-4 | A 61 K 31/715<br>A 61 K 47/48 |
| A | CHEMICAL ABSTRACTS, vol. 96, no. 3, 18 janvier 1982, page 488, abrégé no. 20424b, Columbus, Ohio, US; & JP-A-81 46 838 (MITSUBISHI YUKA YAKUMIN CO., LTD) 28-04-1981<br>* Abrégé *<br>--- | 1-4 | |
| A | CHEMICAL ABSTRACTS, vol. 95, no. 6, 10 août 1981, page 411, abrégé no. 49429x, Columbus, Ohio, US; & JP-A-81 34 618 (ONTA PHARMACEUTICAL CO., LTD) 06-04-1981<br>* Abrégé *<br>----- | 1-4 | |
| | | | **DOMAINES TECHNIQUES RECHERCHES (Int. Cl.5)** |
| | | | A 61 K<br>C 08 B |

Le présent rapport a été établi pour toutes les revendications

| Lieu de la recherche | Date d'achèvement de la recherche | Examinateur |
|---|---|---|
| LA HAYE | 09-07-1991 | BRINKMANN C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)